(19) Europäisches Patentamt
European Patent Office
Office européen des brevets

(11) Veröffentlichungsnummer: **0 000 376**
**B1**

(12) # EUROPÄISCHE PATENTSCHRIFT

(21) Anmeldenummer: **78100317.3**

(22) Anmeldetag: **06.07.78**

(51) Int. Cl.³: **C 07 C 127/00,**
C 07 C 157/02,
A 01 N 47/28

(54) **Neue Harnstoff- und Thioharnstoffverbindungen, Verfahren zu ihrer Herstellung und ihre Verwendung als Fungizide**

(30) Priorität: **12.07.77 JPU 82602/77**

(43) Veröffentlichungstag der Anmeldung:
**24.01.79 Patentblatt 79/02**

(45) Bekanntmachung des Hinweises auf die Patenterteilung:
**20.08.80 Patentblatt 80/17**

(84) Benannte Vertragsstaaten:
**BE CH DE FR GB LU NL**

(56) Entgegenhaltungen:
**NL - A - 7 708 088**

(73) Patentinhaber: **Nihon Tokushu Noyaku Seizo K.K.**
**No. 8, 2-chome, Nihonbashi Muromachi, Chuo-Ku**
**Tokyo (JP)**

(72) Erfinder: **Yamada, Yasuo**
**5 - 15 - 9, Nishihirayama Hino-shi**
**Tokyo (JP)**
**Saito, Junichi**
**3 - 7 - 12, Osawa Mitaka-shi**
**Tokyo (JP)**
**Tamura, Tatsuo**
**2800, Hane, Hamura-cho Nishitama-gun**
**Tokyo (JP)**
**Kurahashi, Yoshio**
**47—15, Oya-machi Hachioji-shi**
**Tokyo (JP)**

(74) Vertreter: **Gremm, Joachim, Dr.**
**Bayer AG c/o Zentralbereich Patente, Marken und Lizenzen Bayerwerk**
**D - 5090 Leverkusen (DE)**

**EP 0 000 376 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

Neue Harnstoff- und Thioharnstoffverbindungen, Verfahren zu ihrer Herstellung und ihre Verwendung als Fungizide

Die Erfindung betrifft neue Harnstoff- und Thioharnstoffverbindungen, ein Verfahren zu ihrer Herstellung und ihre Verwendung als Fungizide.

In der Japanischen Patentveröffentlichung Nr. 29252/1969 ist beschrieben, daß Verbindungen der allgemeinen Formel

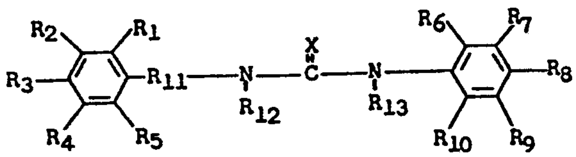

(VI)

worin $R_1$—$R_{10}$, die gleich oder verschieden sein können, jeweils Wasserstoff, Halogen, Niederalkyl, Niederalkoxy oder $NO_2$ bedeuten, mit der Maßgabe, daß wenigstens zwei der Substituenten $R_1$—$R_5$ und wenigstens zwei der Substituenten $R_6$—$R_{10}$ nicht Wasserstoff bedeuten, $R_{11}$ geradkettiges Alkylen bedeutet, $R_{12}$ und $R_{13}$ jeweils Wasserstoff oder Niederalkyl darstellen und X für Sauerstoff oder Schwefel steht, insektizide, akarizide, fungizide und herbizide Wirksamkeit besitzen.

Gemäß der Erfindung werden neue Harnstoff- und Thiorharnstoffverbindungen der allgemeinen Formel

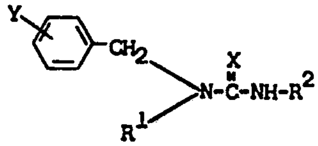

(I)

geschaffen, worin $R^1$ Cycloalkyl mit 5—8 C-Atomen im Ring bedeutet, das gegebenenfalls durch Alkyl mit 1—8 C-Atomen substituiert ist, $R^2$ Alkyl mit 1—8 C-Atomen, Cycloalkyl mit 5—8 C-Atomen im Ring oder Phenyl bedeutet, X für Sauerstoff oder Schwefel steht und Y Halogen, Alkyl mit 1—8 C-Atomen, Cyan oder Nitro darstellt.

Es wurde gefunden, daß die Verbindungen der Formel (I) ausgezeichnete fungizide Wirkung besitzen.

Vorzugsweise bedeutet $R^1$ ein gegebenenfalls durch Alkyl mit 1—4 C-Atomen, insbesondere Methyl, substituiertes Cycloalkyl mit 5—7 C-Atomen im Ring, insbesondere Cyclopentyl oder Cyclohexyl; $R^2$ steht für Äthyl, n-Propyl, n-Butyl, sec.Butyl, Amyl, Cyclopentyl, Cyclohexyl, Cycloheptyl oder Phenyl und Y bedeutet Chlor, Brom, Cyan, Nitro oder Alkyl mit 1—4 C-Atomen, insbesondere Methyl. Besonders bevorzugt steht der Rest Y in 4-Stellung.

Die Erfindung betrifft weiters ein Verfahren zur Herstellung von neuen Harnstoff- oder Thioharnstoffverbindungen der Formel (I), das dadurch gekennzeichnet ist, daß.

a) ein N-Benzyl-N-cycloalkylamin der allgemeinen Formel

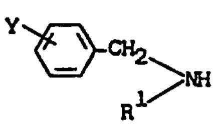

(II)

worin $R^1$ und Y die oben angeführte Bedeutung besitzen, mit einem Isocyanat oder Isothiocyanat der allgemeinen Formel

$$X{=}C{=}N{-}R^2 \qquad (III)$$

worin $R^2$ und X die oben angeführte Bedeutung besitzen, oder

b) ein N-Benzyl-N-cycloalkylcarbamoyl(oder -thiocarbamoyl)-halogenid der allgemeinen Formel

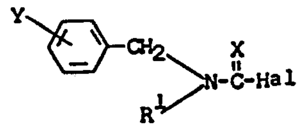

(IV)

worin $R^1$, X und Y die oben angeführte bedeutung haben und Hal Halogen, insbesondere Chlor oder Brom, bedeutet, mit einem Amin der allgemeinen Formel

$$H_2N—R^2 \qquad (V)$$

umgesetzt wird, worin $R^2$ die oben angeführte Bedeutung besitzt.

Beispiele für die als Ausgangsmaterialien gemäß Verfahrensvariante a) einzusetzenden N-Benzyl-N-cycloalkylamine der allgemeinen Formel (II) sind: N - 4 - Methylbenzyl - N - cyclopentylamin, N - 4 - Methylbenzyl - N - 3 - methylcyclopentylamin, N - 4 - Methylbenzyl - N - cyclohexylamin, N - 4 - Methylbenzyl - N - 2 - methylcyclohexylamin, N - 4 - Chlorbenzyl - N - cyclopentylamin, N - 4 - Chlorbenzyl - N - 3 - methylcyclopentylamin, N - 4 - Chlorbenzyl - N - cyclohexylamin, N - 4 - Chlorbenzyl - N - 2 - methylcyclohexylamin, N - 4 - Brombenzyl - N - cyclopentylamin, N - 4 - Brombenzyl - N - 2 - methylcyclohexylamin, N - 4 - Cyanbenzyl - N - cyclopentylamin und N - 4 - Nitrobenzyl - N - chlorpentylamin.

Beispiele für die ebenfalls als Ausgangsstoffe gemäß Verfahrensvariante a) geeigneten Isocyanate und Isothiocyanate der allgemeinen Formel (III) sind: Phenylisocyanat, Phenylisothiocyanat, Cyclopentylisocyanat, Cyclopentylisothiocyanat, Cyclohexylisocyanat, Cyclohexylisothiocyanat, Cycloheptylisocyanat, Cycloheptylisothiocyanat, Äthylisocyanat, Äthylisothiocyanat, Propylisocyanat, Propylisothiocyanat, Butylisocyanat, Butylisothiocyanat, sec.Butylisocyanat, sec.Butylisothiocyanat, Amylisocyanat und Amylisothiocyanat.

Wenn als Ausgangsstoffe gemäß Verfahrensvariante a) N - 4 - Methylbenzyl - N - cyclopentylamin und Phenylisocyanat eingesetzt werden, so kann die Reaktion durch folgendes Schema veranschaulicht werden:

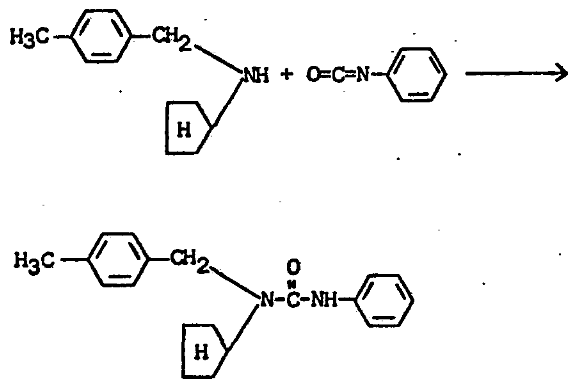

Beispiele für die gemäß Verfahrensvariante b) als Ausgangsstoffe einzusetzenden N - Benzyl - N - cycloalkylcarbamoyl - (und -thiocarbamoyl) - halogenide der allgemeinen Formel (IV) sind N - 4 - Methylbenzyl - N - cyclopentyl-, N - 4 - Methylbenzyl - N - 3 - methylcyclopentyl-, N - 4 - Methylbenzyl - N - cyclohexyl-, N - 4 - Methylbenzyl - N - 2 - methylcyclohexyl-, N - 4 - Chlorbenzyl - N - cyclopentyl-, N - 4 - Chlorbenzyl - N - 3 - methylcyclopentyl-, N - 4 - Chlorbenzyl - N - cyclohexyl-, N - 4 - Chlorbenzyl - N - 2 - methylcyclohexyl-, N - 4 - Brombenzyl - N - cyclopentyl-, N - 4 - Brombenzyl - N - 2 - methylcyclohexyl-, N - 4 - Cyanbenzyl - N - cyclopentyl-, und N - 4 - Nitrobenzyl - N - cyclopentyl - carbamoyl - (oder -thiocarbamoyl) - chloride und die entsprechenden Bromide.

Beispiele für die Amine der allgemeinen Formel (V), die ebenfalls für den Einsatz als Ausgangsstoffe gemäß Verfahrensvariante b) geeignet sind, sind Anilin, Cyclopentylamin, Cyclohexylamin, Cycloheptylamin, Äthylamin, Propylamin, Butylamin, sec.Butylamin und Amylamin.

Wenn gemäß Verfahrensvariante b) 4-N-Chlorbenzyl-N-cyclopentyl-carbamoylchlorid und Cyclohexylamin als Ausgangsstoffe eingesetzt werden, kann die Reaktion durch folgendes Schema veranschaulicht werden:

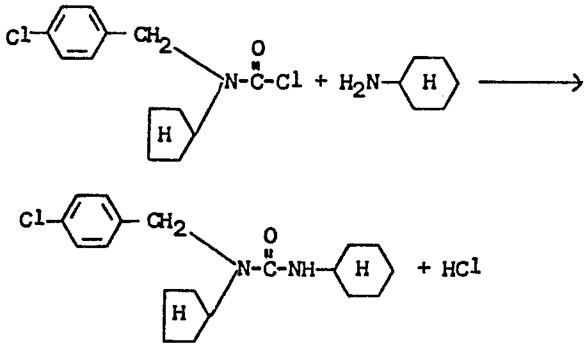

Die Umsetzung gemäß Verfahrensvariante b) kann in Gegenwart eines Säurebindemittels vorgenommen werden. Zu diesem Zweck ist jeder beliebige, herkömmliche Säureakzeptor, wie z.B. Alkalihydroxid, Alkalikarbonat, Alkalibikarbonat, Alkalialkoholat oder tertiäre organische Basen, wie z.B. Triäthylamin, Dimethylanilin oder Pyridin, geeignet.

Die Varianten a) und b) des erfindungsgemäßen Verfahrens werden vorzugsweise in Gegenwart eines Lösungs- und/oder Verdünnungsmittels durchgeführt. Beispiele für geeignete inerte Lösungsmittel oder Verdünnungsmittel sind Wasser und inerte organische Lösungsmittel aus der Gruppe der aliphatischen, alicyclischen und aromatischen Kohlenwasserstoffe, die gegebenenfalls, chloriert sein können, wie z.B. Hexan, Cyclohexan, Petroläther, Ligroin, Benzol, Toluol, Xylol, Methylenchlorid, Chloroform, Tetrachlorkohlenstoff, Äthylenchlorid, Trichloräthylen und Chlorbenzol, Äther, wie z.B. Diäthyläther, Methyläthyläther, Diisopropyläther, Dibutyläther, Propylenoxid, Dioxan und Tetrahydrofuran, Ketone wie z.B. Aceton, Methyläthylketon, Methylisopropylketon und Methylisobutylketon, Nitrile, wie z.B. Acetonitril, Propionitril und Acrylnitril, Alkohole, wie z.B. Methanol, Äthanol, Isopropanol, Butanol und Äthylenglykol, Ester, wie z.B. Äthylacetat und Amylacetat, Säureamide, wie z.B. Dimethylformamid und Dimethylacetamid, Sulfone und Sulfoxide, wie z.B. Dimethylsulfoxid und Dimethylsulfon sowie organische Basen, wie z.B. Pyridin.

Die Varianten a) und b) des erfindungsgemäßen Verfahrens können innerhalb eines weiten Temperaturbereichs durchgeführt werden. Im allgemeinen wird das Verfahren innerhalb eines Temperaturbereichs von —20°C und dem Siedepunkt des Reaktionsgemisches, vorzugsweise bei Temperaturen von 0 bis 100°C, durchgeführt. Die Umsetzung wird vorzugsweise bei Atmosphären-Druck durchgeführt, sie kann jedoch auch bei erhöhtem oder verringertem Druck vorgenommen werden.

Die Verbindungen der Formel (I) besitzen ausgezeichnete fungizide Wirkung und wachstumshemmende Wirksamkeit gegenüber phytopathogenen Pilzen, sie können für die Bekämpfung und Ausrottung verschiedener, durch phytopathogene Pilze hervorgerufener Erkrankungen eingesetzt werden. Sie sind besonders wirksam gegen phytopathogene Pilze aus der Klasse der Basidiomyceten, die z.B. Blattscheiden-Trockenfäule bei Reispflanzen verursachen. Die erfindungsgemäßen Wirkstoffe können gegen parasitäre Pilze, welche den über dem Boden gelegenen Teil der Pflanzen befallen, gegen pathogene Pilze, die die Pflanzen im Erdreich befallen und Tracheomycose verursachen, sowie von Samen und vom Erdreich getragene pathogene Pilze eingesetzt werden.

Die erfindungsgemäßen Wirkstoffe werden vorteilhaft als land- und gartenwirtschaftliche Chemikalien bei der Bekämpfung von Pilzerkrankungen an Pflanzen eingesetzt, da sie nur geringe Toxizität gegenüber Warmblütern und ausgezeichnete Verträglichkeit gegenüber höheren Pflanzen besitzen, d.h. daß sie bei Verwendung in üblichen Konzentrationen keine nachteilige Wirkung auf Kulturpflanzen ausüben.

Die erfindungsgemäßen Verbindungen können daher mit gutem Erfolg als Fungizide bei der Bekämpfung verschiedener, durch pathogene Pilze wie z.B. Archimycetes, Phycomycetes, Ascomycetes, Basidiomycetes und Fungi Imperfecti verursachte Erkrankungen verwendet werden.

Besonders wirksam sind die Wirkstoffe gegenüber Pilzen, welche Trockenfäule bzw. Brand (Pellicularia sasakii) und Sämlingfäule (Pellicularia filamentosa) beides ernstzunehmende Erkrankungen von Reispflanzen verursachen. Außerdem sind die Wirkstoffe nüzlich für die Bekämpfung folgender Krankheiten bei Kulturpflanzen: sklerotische Trockenfäule (Corticium centrifugum), Meltau (Pyricularia oryzae), bakterielle Trockenfäule (Xanthomonas oryzae) an den Blättern der Reispflanzen, bakterielle Weichfäule bei Chinakohl (Erwinia aroideas), Zitrus-Krebs (Rost) (Xanthomonas citri), Grindfäule (Helmintusporium) (Cochliobolus miyabeanus) bei Reispflanzen, Bananenblätter befallende Grind-Fäule (Mycosphaerella musicola), Erdbeeren befallender Grau-Schimmel-Pilz (Botrytis cinerea), Meltau an Weinstöcken (Plasmopara viticola), Anthraknose (Schwarzer Brenner) (Glomella cingulata), bei Wein, Apfelund Birnbäumen, Gemüsepflanzen befallende sklerotische Fäule (Sclerotinia sclerotiorum), Anthraknose bei Melonen (Colletotrichum lagenarium), Melanose (Diaporthe citri) bei Zitrusbäumen, Pulvermeltau bei Apfelbäumen (Podosphaera leucotricha), Pulvermeltau an Gurken (Sphaerotheca fuliginea), Korkflecken auf Äpfeln durch Alternaria mali, Frühfäule der Kartoffelpflanzen durch Alternaria

solani, Schwarzfäule an Birnen durch Alternaria kikuchiana, Apfelschorf (Venturia inaequalis) und Birnenschorf (Venturia pirina).

Aufgrund der vorstehend erwähnten, ausgezeichneten fungiziden Eigenschaften können die erfindungsgemäßen Wirkstoffe mit gutem Erfolg bei der Bekämpfung von durch phytopathogene Pilze verursachten Erkrankungen eingesetzt werden, die bisher mit Fungiziden bekämpft wurden, welche Schwermetalle, Arsen oder Quecksilber enthalten und für Mensch und Tier schädlich sind.

Die erfindungsgemäßen Wirkstoffe können in die üblichen Formulierungen wie Lösungen, Emulsionen, benetzbare Pulver, Suspensionen, Pulver, Stäube, Schäume, Pasten, lösliche Pulver, Granulate, Aerosole, konzentrierte Suspensionen und Emulsionen, Samen-Behandlungspulver, natürliche und synthetische, mit dem Wirkstoff imprägnierte Materialien, sehr feine Kapseln in polymeren Substanzen und Beschichtungskompositionen für den Einsatz auf Samen und Formulierungen für den Einsatz mittels Räucherausrüstungen wie z.B. Verräucherungspatronen, -kanister und -schlangen, sowie in Ultra-Low-Volume-(ULV)-Kaltnebel und Warmnebel-Formulierungen übergeführt werden.

Die Formulierungen können nach bekannten Verfahren wie z.B. durch Vermischen des Wirkstoffs mit Streckmitteln, d.h. flüssigen oder verflüssigten gasförmigen oder festen Verdünnungs- oder Trägermitteln, gegebenenfalls unter Verwendung von oberflächenaktiven Mitteln, d.h. Emulgatoren und/oder Dispergiermitteln und/oder Schaumbildnern hergestellt werden. Bei Verwendung von Wasser als Sreckmittel können organische Lösungsmittel als Hilfsstoffe eingesetzt werden.

Als Beispiele für flüssige Verdünnungs- oder Trägermittel, insbesondere Lösungsmittel, sind vor allem aromatische Kohlenwasserstoffe wie z.B. Xylol, Toluol oder Alkylnaphthaline, chlorierte aromatische oder chlorierte aliphatische Kohlenwasserstoffe, wie z.B. Chlorbenzole, Chloräthylene oder Methylenchlorid, aliphatische oder alicyclische Kohlenwasserstoffe, wie z.B. Cyclohexan, oder Paraffine, wie z.B. Erdölfraktionen Alkohole, wie z.B. Butanol oder Glykol und deren Äther und Ester, Ketone, wie z.B. Aceton, Methyläthylketon, Methylisobutylketon oder Cyclohexanon, oder stark polare Lösungsmittel, wie z.B. Dimethylformamid und Dimethylsulfoxid sowie Wasser zu nennen.

Unter verflüssigten gasförmigen Verdünnungs- oder Trägermitteln sind Flüssigkeiten zu verstehen, die bei normalen Temperaturen und formalen Drucken gasförmig sind, wie z.B. Aerosoltreibmittel, z.B. halogenierte Kohlenwasserstoffe, und Butan, Propan, Stickstoff und Kohlendioxid.

Als feste Trägermittel sind vermahlene, natürliche Mineralien, wie z.B. Kaoline, Tone, Talk, Kreide, Quarz, Attapulgit, Montmorillonit oder Kieselgur und vermahlene synthetische Mineralien, wie z.B. hochdisperse Kieselsäure, Aluminiumoxid und Silikate, geeignet. Als feste Trägermittel für Granulate können zerkleinerte und fraktionierte natürliche Gesteine, wie z.B. Kalzit, Marmor, Bimsstein, Sepiolit und Dolomit, sowie synthetische Granulate aus anorganischen und organischen Mehlen und Granulate aus organischen Materialien wie z.B. Sägespänen bzw. Sägemehl, Kokosnußschalen, Maiskolben und Tabakstengeln, verwendet werden.

Beispiele für geeignete Emulgatoren und/oder Sohaumbildner sind nichtionische und anionische Emulgatoren, wie z.B. Polyoxyäthylenfettsäureester, Polyoxyäthylenfettalkoholäther, wie z.B. Alkylarylpolyglykoläther, Alkylsulfonate, Alkylsulfate, Arylsulfonate sowie Albuminhydrolyseprodukte. Als geeignete Dispergiermittel sind Ligninsulfitablaugen und Methylcellulose zu nennen.

In den Formulierungen können Kleber wie Carboxymethylzellulose und natürliche und synthetische Polymer in Form von Pulvern, Granulaten oder Latices, wie z.B. Gummi arabicum, Polyvinylalkohol und Polyvinylacetat, eingesetzt werden.

Es können Färbemittel, wie z.B. anorganische Pigmente, wie z.B. Eisenoxid, Titanoxid und Preußischblau und organische Farbstoffe, wie z.B. Alizarinfarbstoffe, Azofarbstoffe oder Metallphthalcyaninfarbstoffe und Spuren-Nährstoffe, wie z.B. die Salze von Eisen, Mangen, Bor, Kupfer, Kobalt, Molybdän und Zink, verwendet werden.

Die erfindungsgemäßen Wirkstoffe können in den Formulierungen vermischt mit anderen Wirkstoffen, wie z.B. Fungiziden, Insektiziden, Akariziden, Nematoziden, Herbiziden, Abschreckungsmitteln für Vögel, Wachstumsregulatoren, Pflanzennährstoffen und Bodenstrukturverbesserungsmitteln eingesetzt werden.

Die Formulierungen enthalten im allgemeinen 0,1—95 Gew.-%, vorzugsweise 0,5—90 Gew.-%, an Wirkstoff.

Die Wirkstoffe können so, wie sie sind, oder in Form von aus den Formulierungen durch weitere Verdünnung hergestellten gebrauchsfertigen Lösungen, Emulsionen, Suspensionen, Pulvern, Pasten und Granulaten eingesetzt werden. Auch ihre Anwendung kann auf übliche Weise z.B. durch Bewässern, Versprühen, Zerstäuben, Bestäuben, Verstreuen, Trockenbeizen, Feuchtbeizen und Naßbeizen, Schlammbeizen oder Inkrustieren erfolgen.

Die Wirkstoffkonzentrationen in den Gebrauchs-Formen können innerhalb eines weiten Bereiches variiert werden. Sie liegen im allgemeinen zwischen 0,0001 und 20 Gew.-%, vorzugsweise von 0,005 bis 10 Gew.-%.

Im allgemeinen werden 0,03—10 kg, vorzugsweise 0,3—6 kg, Wirkstoff pro Hektar Bodenfläche eingesetzt.

Die vorliegende Erfindung schafft auch eine fungizide Komposition, die als Wirkstoff eine erfindungsgemäße Verbindung (I) vermischt mit einem festen oder verflüssigten, gasförmigen Verdünnungs- oder Trägermittel oder vermischt mit einem flüssigen, ein oberflächenaktives Mittel enthaltenden Verdünnungs- oder Trägermittel enthält.

Die Erfindung betrifft weiters die Verwendung der Verbindungen der Formel I zur Bekämpfung von Pilzen; dabei wird auf die Pilze oder deren Lebensraum eine erfindungsgemäße Verbindung allein oder in Form einer Komposition, die als Wirkstoff eine erfindungsgemäße Verbindung vermischt mit einem Verdünnungs- oder Trägermittel enthält, aufgebracht.

Acker- und Gartenbauprodukte können vor Pilzbefall dadurch geschützt werden, daß sie in Gebieten gezogen werden, auf welche unmittelbar vor und/oder während des Wachstums der Pflanzen eine erfindungsgemaße Verbindung der allgemeinen Formel (I) allein oder vermischt mit einem Verdünnungs- oder Trägermittel aufgebracht worden ist.

Die üblichen Verfahren zur Erzeilung von Ernten an Acker- und Gartenbau-Produkten können durch die vorliegende Erfindung verbessert werden.

Die Erfindung wird an Hand der nachstehenden Beispiele näher erläutert. Die erfindungsgemäßen Verbindungen werden jeweils mit der Zahl des entsprechenden Herstellungsbeispiels bezeichnet. Teile bedeuten Gewichtsteile.

Beispiel (i) (Benetzbares Pulver)

50 Gew.-Teile der Verbindung Nr. 1, 45 Gew.-Teile eines Gemisches (1:5) aus Kieselgur und Kaolin und 5 Teile eines Emulgators (Polyoxyäthylenalkylphenyläther) werden pulverisiert und zu einem benetzbaren Pulver vermischt, das vor dem Aufsprühen mit Wasser auf eine Konzentration von 0,05% verdünnt wird.

Beispiel (ii) (Emulgierbares Konzentrat)

30 Teile der Verbindung Nr. 3, 30 Teile Xylol, 30 Teile Methylnaphthalin und 10 Teile eines Polyoxyäthylenalkylphenyläthers werden unter Rühren in ein emulgierbares Konzentrat übergeführt, das vor dem Versprühen mit Wasser auf eine Konzentration von 0,05% verdünnt wird.

Beispiel (iii) (Staub)

2 Teile der Verbindung Nr. 21 und 98 Teile eines Gemisches (1:3) aus Talk und Ton werden pulverisiert und zu einem Staub vermischt, der verstäubt wird.

Beispiel (iv) (Staub)

1,5 Teile der Verbindung Nr. 23, 0,5 Teile Isopropylhydrogenphosphat und 98 Teile eines Gemisches (1:3) aus Talk und Ton werden pulverisiert und zu einem Staub vermischt.

Beispiel (v) (Granulat)

10 Teile der Verbindung Nr. 27, 10 Teile Bentonit, 78 Teile eines Gemisches (1:3) aus Talk und Ton und 2 Teile Lighninsulfonat werden vermischt und mit 25 Teilen Wasser versetzt. Das gründlich vermengte Gemisch wird mittels Extrusionsgranulator zu einem Granulat mit einer Teilchengröße von 350—700 $\mu$m (20—40 mesh) verarbeitet, das bei 40—50°C getrocknet wird.

Beispiel (vi) (Granulat)

95 Teile Tonpulver mit einer Korngrößenverteilung von 0,2 bis 2 mm werden in einen Rotationsmischer eingebracht. Während der Rotation werden die Teilchen gleichmäßig mit einer Lösung von 5 Teilen der Verbindung Nr. 29 in einem organischen Lösungsmittel besprüht. Durch Trocknen bei 40—50°C wird die Mischung in ein Granulat übergeführt.

Beispiel (vii) (Öliges Präparat)

0,5 Teile der Verbindung Nr. 30, 20 Teile einer hochsiedenden aromatischen Verbindung und 79,5 Teile Kerosin werden unter Rühren zu einem öligen Präparat vermischt.

Die fungizide Wirksamkeit der erfindungsgemäßen Verbindungen ist aus dem nachstehenden Biotestbeispiel ersichtlich.

In diesem Beispiel werden die erfindungsgemäßen Verbindungen jeweils mit der (in Klammern angeführten) Zahl des entsprechenden Herstellungsbeispiels bezeichnet, das später in der Beschreibung folgt.

Die bekannten Vergleichsverbindungen werden wie folgt definiert:

(A) = 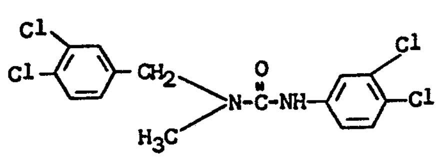

(in der japanischen Patentveröffentlichung Nr. 29252/1969 beschrieben)

(B) = ein Polyoxinkomplex (handelsüblich)
(C) = Bis-(dimethylthiocarbamoylthio)-methyl-arsin

Beispiel A
Test auf Wirksamkeit gegen Pellicularia sasakii (Blattscheidentrockenfäule), Topftest.
Zubereitung der fungiziden Komposition:

| | |
|---|---|
| Wirkstoff: | 50 Gew.-Teile |
| Trägermittel: | 45 Gew.-Teile eines Gemisches (1:5) aus Kieselgur und Kaolin |
| Emulgator: | 5 Gew.-Teile Polyoxyäthylenalkylphenyläther |

Die vorstehend angeführten Mengen Wirkstoff, Trägermittel und Emulgator wurden zu einem benetzbaren Pulver vermahlen, welches mit Wasser auf die vorgeschriebene Konzentration verdünnt wurde.

Testverfahren:

Reispflanzen (Varietät: Kinmaze) wurden in Wagnertöpfen (0,0002 a) unter Überschwemmungsbedingungen gezogen. Wenn die Reispflanze das frühe Ährenstadium erreicht hatte, wurde ein flüssiges Präparat, das wie oben beschrieben hergestellt worden war und einen Wirkstoff in vorgeschriebener Konzentration enthielt, in Mengen von 100 ml/pro 3 Töpfe auf diese aufgebracht.

Einen Tag nach dem Aufbringen des Wirkstoffs wurden die unteren Teile der Pflanzen mit dem Fungus Pellicularia sasakii (der 10 Tage lang auf einem Gerstennährboden gezogen worden war, un Sklerotien zu bilden), beimpft. Die Pflanzen wurden in einem Gewächshaus bei 28—30°C und einer relativen Luftfeuchtigkeit von mindestens 95% gehalten. Dann wurde der Befallsgrad bewertet und die Phytotoxizität des Wirkstoffs bestimmt. Bei der Bewertung wurde die Ausdehnung der befallenen Teile vom Beimfungspunkt (am unteren Teil der Pflanzen) aus bestimmt und wie folgt berechnet:

$$\text{Befallsgrad: } \frac{3n_3 + 2n_2 + n_1 + n_0}{3\ N} \times 100$$

worin

N die Gesamtzahl der untersuchten Pflanzenstengel,
$n_0$ die Zahl der nicht befallenen Stengel,
$n_1$ die Zahl der Stengel, an welchen sich der Befall vom unteren Teil der Pflanze bis zum ersten Blattscheidenabschnitt erstreckt,
$n_2$ die Zahl der Stengel, an welchen sich der Befall vom unteren Teil der Pflanze bis zum zweiten Blattscheidenabschnitt erstreckt und
$n_3$ die Anzahl der Stengel bedeutet, an welchem sich der Befall vom unteren Teil der Pflanze bis zum dritten Blattscheidenabschnitt erstreckt.

Die Ergebnisse sind aus Tabelle A ersichtlich. Das Symbol "-" in der letzten Spalte bedeutet, daß keine Phytotoxizität beobachtet wurde.

TABELLE A

Ergebnisse des Tests auf Wirksamkeit gegen Pellicularia sasakii

| Wirkstoff | Wirkstoff-konzentration in % | Befallsgrad | Phyto-toxizitat |
|---|---|---|---|
| (1) | 0,0125 | 0 | – |
| | 0,025 | 0 | – |
| | 0,05 | 0 | – |
| (2) | 0,0125 | 0 | – |
| | 0,025 | 0 | – |
| | 0,05 | 0 | – |
| (3) | 0,0125 | 5,3 | – |
| | 0,025 | 0 | – |
| | 0,05 | 0 | – |
| (4) | 0,05 | 11,5 | – |
| | 0,1 | 2,3 | – |
| (5) | 0.0125 | 7,3 | – |
| | 0,025 | 0,5 | – |
| | 0,05 | 0 | – |
| (6) | 0,025 | 9,8 | – |
| | 0,05 | 0 | – |
| (7) | 0,025 | 5,2 | – |
| | 0,05 | 0 | – |
| (8) | 0,05 | 20,0 | – |
| | 0,1 | 10,4 | – |
| (9) | 0,05 | 18,5 | – |
| | 0,1 | 9,8 | – |
| (10) | 0,05 | 13,3 | – |
| | 0,1 | 5,7 | – |
| (11) | 0,05 | 7,3 | – |
| | 0,1 | 0 | – |
| (12) | 0,0125 | 11,2 | – |
| | 0,025 | 0 | – |
| | 0,05 | 0 | – |
| (13) | 0,0125 | 9,4 | – |
| | 0,025 | 0 | – |
| | 0,05 | 0 | – |
| (14) | 0,025 | 9,0 | – |
| | 0,05 | 3,5 | – |
| (15) | 0,05 | 12,3 | – |
| | 0,1 | 7,7 | – |
| (16) | 0,05 | 10,9 | – |
| | 0,1 | 4,3 | – |

TABELLE A

| Wirkstoff | Wirkstoff-konzentration in % | Befallsgrad | Phyto-toxizität |
|---|---|---|---|
| (17) | 0,0125 | 3,5 | – |
| | 0,025 | 0 | – |
| | 0,05 | 0 | – |
| (18) | 0,025 | 7,3 | – |
| | 0,05 | 0,5 | – |
| (19) | 0,0125 | 9,5 | – |
| | 0,025 | 0,5 | – |
| | 0,05 | 0 | – |
| (20) | 0,025 | 7,8 | – |
| | 0,05 | 0,7 | – |
| (21) | 0,0125 | 0 | – |
| | 0,025 | 0 | – |
| | 0,05 | 0 | – |
| (22) | 0,0125 | 4,3 | – |
| | 0,025 | 0 | – |
| | 0,05 | 0 | – |
| (23) | 0,0125 | 0 | – |
| | 0,025 | 0 | – |
| | 0,05 | 0 | – |
| (24) | 0,05 | 15,5 | – |
| | 0,1 | 7,5 | – |
| (25) | 0,05 | 18,9 | – |
| | 0,1 | 10,0 | – |
| (27) | 0,025 | 10,3 | – |
| | 0,05 | 4,1 | – |
| (28) | 0,0125 | 0 | – |
| | 0.025 | 0 | – |
| | 0,05 | 0 | – |
| (29) | 0,0125 | 0 | – |
| | 0,025 | 0 | – |
| | 0,05 | 0 | – |
| (30) | 0,0125 | 0 | – |
| | 0,025 | 0 | – |
| | 0,05 | 0 | – |
| (31) | 0,0125 | 3,7 | – |
| | 0,025 | 0 | – |
| | 0,05 | 0 | – |
| (32) | 0,0125 | 5,3 | – |
| | 0,025 | 0 | – |
| | 0,05 | 0 | – |
| (33) | 0,0125 | 5,3 | – |
| | 0,025 | 0 | – |
| | 0,05 | 0 | – |

TABELLE A

| Wirkstoff | Wirkstoff-konzentration in % | Befallsgrad | Phyto-toxizität |
|---|---|---|---|
| (34) | 0,0125 | 4,7 | – |
| | 0,025 | 0 | – |
| | 0,05 | 0 | – |
| (35) | 0,0125 | 0 | – |
| | 0,025 | 0 | – |
| | 0,05 | 0 | – |
| (36) | 0,025 | 8,7 | – |
| | 0,05 | 0 | – |
| (37) | 0,0125 | 0 | – |
| | 0,025 | 0 | – |
| | 0,05 | 0 | – |
| (38) | 0,025 | 10,3 | – |
| | 0,05 | 0,5 | – |
| (39) | 0,0125 | 0 | – |
| | 0,025 | 0 | – |
| | 0,05 | 0 | – |
| (40) | 0,025 | 12,5 | – |
| | 0,05 | 1,0 | – |
| (41) | 0,0125 | 0 | – |
| | 0,025 | 0 | – |
| | 0,05 | 0 | – |
| (42) | 0,025 | 3,7 | – |
| | 0,05 | 0 | – |
| (43) | 0,0125 | 0 | – |
| | 0,025 | 0 | – |
| | 0,05 | 0 | – |
| (44) | 0,0125 | 0 | – |
| | 0,025 | 0 | – |
| | 0,05 | 0 | – |
| (45) | 0.025 | 14,7 | – |
| | 0,05 | 9,4 | – |
| (46) | 0,0125 | 3,6 | – |
| | 0,025 | 0 | – |
| | 0,05 | 0 | – |
| (47) | 0,0125 | 0 | – |
| | 0,025 | 0 | – |
| | 0,05 | 0 | – |
| (48) | 0,0125 | 0 | – |
| | 0,025 | 0 | – |
| | 0,05 | 0 | – |
| (49) | 0,05 | 7,3 | – |
| | 0,1 | 0 | – |

TABELLE A

| Wirkstoff | Wirkstoff-konzentration in % | Befallsgrad | Phyto-toxizität |
|---|---|---|---|
| (50) | 0,0125 | 0 | – |
| | 0,025 | 0 | – |
| | 0,05 | 0 | – |
| (51) | 0,0125 | 0 | – |
| | 0,025 | 0 | – |
| | 0,05 | 0 | – |
| (A) | 0,05 | 73,5 | – |
| | 0,1 | 53,0 | – |
| (B) | 0,0045 | 25,7 | – |
| (C) | 0,008 | 2,3 | ± |
| Kontrolle | – | 75,4 | – |

Das Verfahren zur Herstellung der erfindungsgemäßen Verbindungen wird an Hand der nachstehenden Herstellungsbeispiele näher erläutert.

Beispiel 1

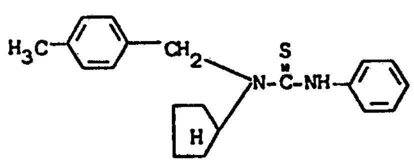

(1)

Unter Kühlung und Rühren wurde eine Lösung von 14 g Phenylisothiocyanat in 50 ml Hexan zu 19 g N-4-Methylbenzyl-N-cyclopentylamin in 400 ml Hexan zugetropft. Nach beendeter Zugabe wurde die Temperatur des Reaktionsgemisches allmählich erhöht, die Lösung wurde bei 40°C etwa 5 Stunden lang gerührt. Das Gemisch wurde sodann gekühlt und filtriert. Umkristallisieren aus einem Gemisch von Hexan und Äthylalkohol ergab 30 g N-4-Methylbenzyl-N-cyclopentyl-N'-phenylthioharnstoff mit einem Schmelzpunkt von 109—111°C.

Beispiel 2

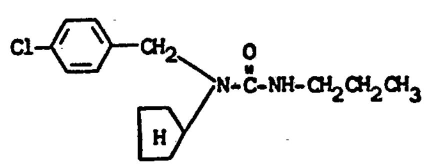

(2)

Analog zu der in Beispiel 1 beschriebenen Methode wurden 31 g N-4-Chlorbenzyl-N-cyclopentylamin mit 13 g Propylisocyanat umgesetzt und es wurden 32 g N-4-Chlorbenzyl-N-cyclopentyl-N'-propylharnstoff mit einem Schmelzpunkt von 103—105°C erhalten.

Andere gemäß den in den Beispielen 1 und 2 beschriebenen Methoden hergestellte, erfindungsgemäße Verbindungen sind in Tabelle 1 angeführt.

TABELLE 1

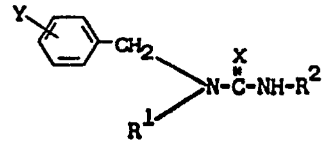

(I)

| Beispiel Nr. | $R^1$ | $R^2$ | X | Y | Brechungsindex oder Schmelzpunkt in °C |
|---|---|---|---|---|---|
| 3 | 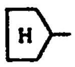 | 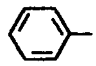 | O | 4–$CH_3$ | 125 – 127 |
| 4 | 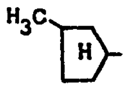 | 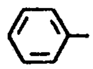 | O | 4–$CH_3$ | 124 – 125 |
| 5 | 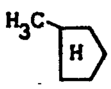 | 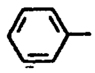 | S | 4–$CH_3$ | 55 – 63 |
| 6 | 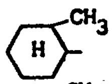 | 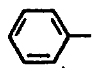 | O | 4–$CH_3$ | 138 – 139 |
| 7 | 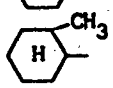 | 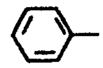 | S | 4–$CH_3$ | 141 – 146 |
| 8 | 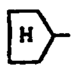 | 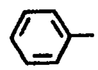 | O | 4–$CH_3$ | 159 – 160 |
| 9 | 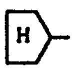 | 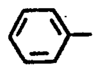 | S | 4–CN | 85 – 90 |
| 10 | 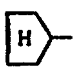 | 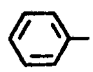 | O | 4–$NO_2$ | 168 – 171 |
| 11 | 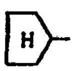 | 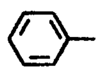 | S | 4–$NO_2$ | 124 – 129 |
| 12 | 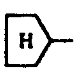 | 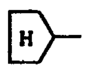 | O | 4–Cl | 91 – 92 |
| 13 | 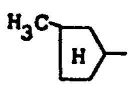 | 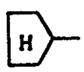 | O | 4–Cl | 63 – 73 |
| 14 | 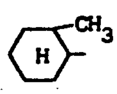 | 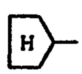 | O | 4–Cl | 131 – 133 |
| 15 | 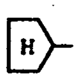 | 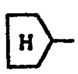 | O | 4–Br | 95 – 96 |
| 16 | 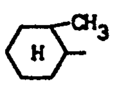 | 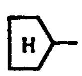 | O | 4–Br | 127 – 128 |
| 17 | 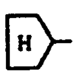 | 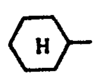 | O | 4–Cl | 99 – 101 |
| 18 | 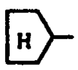 | 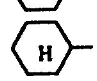 | S | 4–Cl | 115 – 120 |
| 19 | 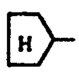 | 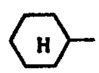 | O | 4–Br | 107 – 108 |

TABELLE 1

| Beispiel Nr. | $R^1$ | $R^2$ | X | Y | Brechungsindex oder Schmelzpunkt in °C |
|---|---|---|---|---|---|
| 20 | 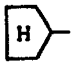 | 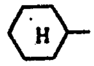 | S | 4–Br | 123 – 124 |
| 21 | 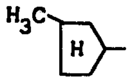 | 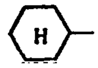 | O | 4–Cl | 86 – 88 |
| 22 | 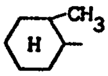 | 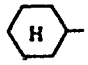 | O | 4–Cl | 147 – 148 |
| 23 | 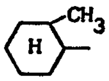 | 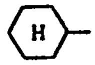 | S | 4–Cl | 135 – 136 |
| 24 | 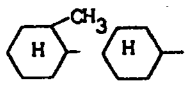 | | O | 4–Br | 140 – 141 |
| 25 | 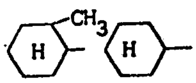 | | S | 4–Br | 131 – 133 |
| 26 | 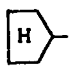 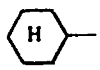 | | O | 4–Cl | 81 – 82 |
| 27 | 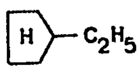 | | O | 4–Cl | 128 – 131 |
| 28 | 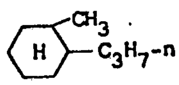 | | O | 4–Cl | 85 – 86 |
| 29 | 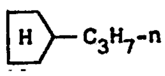 | | O | 4–Br | 96 – 97 |
| 30 | 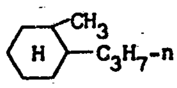 | | O | $4–CH_3$ | 47 – 54 |
| 31 | 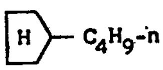 | | O | 4–Cl | 69 – 72 |
| 32 | 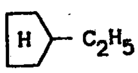 | | O | 4–Br | 114 – 115 |
| 33 | 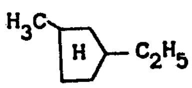 | | O | 4–Cl | 88 – 91 |
| 34 | 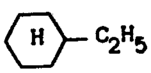 | | O | 4–Cl | 88 – 89 |
| 35 | 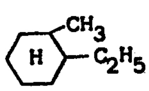 | | O | 4–Cl | 95 – 98 |
| 36 | 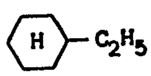 | | O | $4–CH_3$ | 77 – 79 |
| 37 | 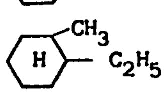 | | O | $4–CH_3$ | 62 – 64 |
| 38 | 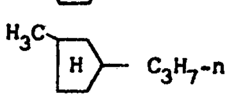 | | O | 4–Cl | 82 – 84 |

TABELLE 1

| Beispiel Nr. | R¹ | R² | X | Y | Brechungsindex oder Schmelzpunkt in °C |
|---|---|---|---|---|---|
| 39 | 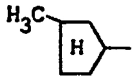 | $C_3H_7$-n | O | 4–$CH_3$ | $n_D^{20}$ = 1,5255 |
| 40 | 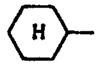 | $C_3H_7$-n | O | 4–$CH_3$ | 75 – 76 |
| 41 | 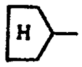 | $C_4H_9$-n | O | 4–Br | 65 – 67 |
| 42 | 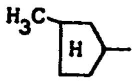 | $C_4H_9$-n | O | 4–Cl | 53 – 58 |
| 43 | 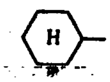 | $C_4H_9$-n | O | 4–Cl | 88 – 89 |
| 44 | 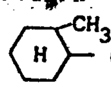 | $C_4H_9$-n | O | 4–Cl | 99 – 101 |
| 45 | 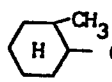 | $C_4H_9$-n | O | 4–$CH_3$ | 40 – 53 |
| 46 | 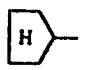 | $C_4H_9$-sec | O | 4–Cl | 99 – 103 |
| 47 | 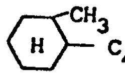 | $C_4H_9$-sec | O | 4–Cl | 120 – 125 |
| 48 | 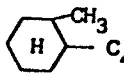 | $C_4H_9$-sec | O | 4–Br | 130 – 133 |
| 49 | 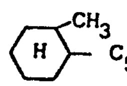 | $C_5H_{11}$-n | O | 4–Cl | 119 – 120 |
| 50 | 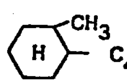 | $C_4H_9$-n | S | 4–Cl | 143 – 145 |
| 51 | 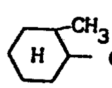 | $C_4H_9$-n | S | 4–$CH_3$ | 101 – 103 |

Beispiel 17 (Alternativverfahren)

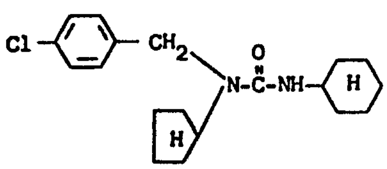

(17)

Eine Lösung aus 27 g N-4-Chlorbenzyl-N-cyclopentyl-carbamoylchlorid in 100 ml Toluol wurde zu 20 g Cyclohexylamin in 400 ml Toluol unter Kühlung und Rühren zugetropft. Nach beendeter Zugabe wurde die Temperatur des Reaktionsgemisches allmählich erhöht und das Gemisch bei 70—80°C etwa 10 Stunden lang gerührt. Nach dem Abkühlen wurde das entstandene Cyclohexylaminhydrochlorid abfiltriert. Die Toluolschicht wurde mit Wasser, 1%igem, wässerigem Natriumcarbonat, 1%iger Salzsäure und Wasser gewaschen und uber wasserfreiem Natriumsulfat getrocknet. Danach wurde das Toluol abdestilliert und der Rückstand aus einem Gemisch von Hexan und Äthylalkohol umkristallisiert, und es wurden 25 g N-4-Chlorbenzyl-N-cyclopentyl-N'-cyclohexylharnstoff mit einem Schmelzpunkt von 99—101°C erhalten.

**Patentansprüche**

1. Harnstoff- und Thioharnstoffverbindungen der allgemeinen Formel

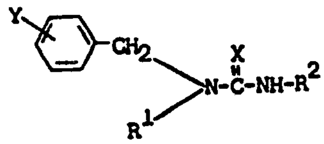

(I),

worin $R^1$ Cycloalkyl mit 5—8 C-Atomen im Ring bedeutet, das gegebenenfalls durch Alkyl mit 1—8 C-Atomen substituiert ist, $R^2$ Alkyl mit 1—8 C-Atomen, Cycloalkyl mit 5—8 C-Atomen im Ring oder Phenyl bedeutet, X für Sauerstoff oder Schwefel steht und Y Halogen, Alkyl mit 1—8 C-Atomen, Cyan oder Nitro darstellt.

2. Verfahren zur Herstellung von neuen Harnstoff- oder Thioharnstoffverbindungen der Formel (I), dadurch gekennzeichnet, daß
a) ein N-Benzyl-N-cycloalkylamin der allgemeinen Formel

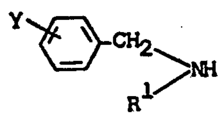

(II)

worin $R^1$ und Y die im Anspruch 1 angeführte Bedeutung besitzen, mit einem Isocyanat oder Isothiocyanat der allgemeinen Formel

$$X{=}C{=}N{-}R^2 \qquad (III)$$

worin $R^2$ und X die im Anspruch 1 angeführte Bedeutung besitzen, oder
b) ein N-Benzyl-N-cycloalkylcarbamoyl(oder -thiocarbamoyl)-halogenid der allgemeinen Formel

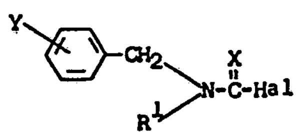

(IV),

worin $R^1$, X und Y die im Anspruch 1 angeführte Bedeutung haben und Hal Halogen, insbesondere Chlor oder Brom, bedeutet, mit einem Amin der allgemeinen Formel

$$H_2N{-}R^2 \qquad (V)$$

umgesetzt wird, worin $R^2$ die im Anspruch 1 angeführte Bedeutung besitzt.

3. Fungizides Mittel, gekennzeichnet durch einen Gehalt an Harnstoff- und Thioharnstoffverbindungen gemäß Anspruch 1.

4. Verwendung von Harnstoff- und Thioharnstoffverbindungen gemäß Anspruch 1 zur Bekämpfung von Pilzen.

5. Verfahren zur Herstellung von fungiziden Mitteln, dadurch gekennzeichnet, daß man Harnstoff- und Thioharnstoffverbindungen gemäß Anspruch 1 mit Streckmitteln und/oder oberflächenaktiven Mitteln vermischt.

**Claims**

1. Urea and thiourea compounds of the general formula

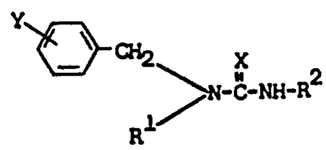

(I)

in which $R^1$ represents cycloalkyl with 5—8 carbon atoms in the ring, which may be optionally substituted by alkyl with 1—8 carbon atoms, $R^2$ represents alkyl with 1—8 carbon atoms, cycloalkyl with 5—8 carbon atoms in the ring or phenyl, X represents oxygen or sulphur, and Y represents halogen, alkyl with 1—8 carbon atoms, cyano or nitro.

2. A process for the preparation of new urea or thiourea compounds of formula (I), characterised in that a) an N-benzyl-N-cycloalkylamine of the general formula

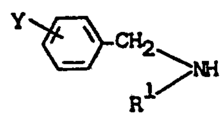

(II)

in which $R^1$ and Y have the meanings stated in claim 1, is reacted with an isocyanate or isothiocyanate of the general formula

$$X{=}C{=}N{-}R^2 \quad (III)$$

in which $R^2$ and X have the meanings stated in claim 1, or (b) an N-benzyl-N-cycloalkyl carbamoyl (or thiocarbamoyl) halide of the general formula

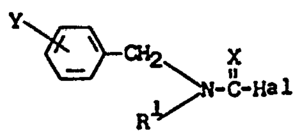

(IV)

in which $R^1$, X and Y have the meanings stated in claim 1, and Hal represents halogen, in particular chlorine or bromine, is reacted with an amine of the general formula

$$H_2N{-}R^2 \quad (V),$$

in which $R^2$ has the meaning stated in claim 1.

3. A fungicidal composition, characterised in that it contains urea and thiourea compounds according to claim 1.

4. The use of urea and thiourea compounds according to claim 1 for combating fungi.

5. A process for the preparation of fungicidal compositions, characterized in that urea and thiourea compounds according to claim 1 are mixed with diluents and surface-active ingredients.

**Revendications**

1. Dérivés d'urée ou de thiourée de formule générale

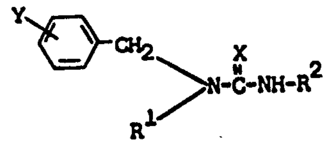

(I)

dans laquelle $R^1$ représente un groupe cycloalkyle en $C_5$—$C_8$, éventuellement substitué par un groupe alkyle en $C_1$—$C_8$, $R^2$ est un groupe alkyle en $C_1$—$C_8$, cycloalkyle en $C_5$—$C_8$ dans le noyau ou phényle, X représente l'oxygène ou le soufre et Y un halogène, un groupe alkyle en $C_1$—$C_8$, cyano ou nitro.

2. Procédé pour la préparation de nouveaux dérivés d'urée ou de thiourée de formule (I), caractérisé en ce que l'on fait réagir:

a) une N-benzyl-N-cycloalkylamine de formule générale

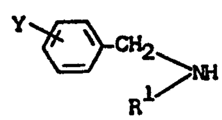

(II)

dans laquelle $R^1$ et Y ont la signification indiquée dans la revendication 1, avec un isocyanate ou isothiocyanate de formule générale

$$X{=}C{=}N{-}R^2 \quad (III)$$

dans laquelle $R^2$ et X ont la signification indiquée dans la revendication 1, ou bien

b) un halogénure de N-benzyl-N-cycloalkylcarbamoyle (ou thiocarbamoyle) de formule générale

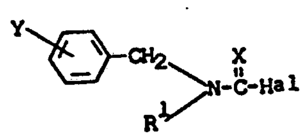

(IV)

dans laquelle $R^1$, X et Y ont la signification indiquée dans la revendication 1 et Hal représente un halogène, en particulier le chlore ou le brome, avec une amine de formule générale

$$H_2N{-}R^2 \quad (V)$$

où $R^2$ a la signification indiquée dans la revendication 1.

3. Agent fongicide, caractérisé en ce qu'il contient des dérivés d'urée ou de thiourée selon la revendication 1.

4. Utilisation des dérivés d'urée et de thiourée selon la revendication 1 pour la lutte contre les champignons.

5. Procédé pour la préparation d'agent fongicide, caractérisé en ce que l'on mélange des dérivés d'urée et de thiourée selon la revendication 1 avec des agents diluants et/ou des agents tensioactifs.